# EUROPEAN PATENT APPLICATION

(11) **EP 2 266 568 A1**
(43) Date of publication of application: **29.12.2010**
(21) Application number: 09161076.6
(22) Date of filing: 26.05.2009
(51) Int. Cl.: A61K 31/4985, A61K 31/519, A61K 38/09, A61P 13/08, A61P 15/00

(54) **Use of LHRH antagonists in combination with PDE V inhibitors for the treatment of sex hormone dependent disorders**

(71) Applicant: Æterna Zentaris GmbH, 60314 Frankfurt am Main (DE)
(72) Inventor: Engel, Jürgen, 63755, Alzenau (DE); Günther, Eckard, 63477 Maintal (DE); Müller, Markus, 60594 Frankfurt am Main (DE)

(57) **Abstract**

The present invention relates to methods of treatment of sex hormone dependent disorders, including genitourinary disorders, infertility and sexual dysfunctions, in which LHRH antagonists are used in combination with PDE V inhibitors. It particulary refers to the treatment of benign prostatic hypertrophy (BPH) and lower urinary tract symptoms (LUTS).

It further relates to pharmaceutical compositions comprising LHRH antagonists in combination with PDE V inhibitors for use in the treatment of sex hormone dependent disorders.

## Description

### FIELD OF THE INVENTION

The present invention relates to methods of treatment of sex hormone dependent disorders, including genitourinary disorders, infertility and sexual dysfunctions, in which LHRH antagonists are used in combination with PDE V inhibitors.

It further relates to pharmaceutical compositions comprising LHRH antagonists in combination with PDE V inhibitors for use in the treatment of sex hormone dependent disorders.

### BACKGROUND OF THE INVENTION

Sex hormone dependent disorders, including genitourinary disorders, infertility and sexual dysfunctions present one of the most serious threats to men's and women's health today.

Benign prostatic hypertrophy (BPH) is a disease conditioned by age and affects approximately 60% of all men older than 60. Pathogenetically, an elevated accumulation of dihydrotestosterone in the prostate tissue is assumed to cause enlargement of the prostate. The accumulation of dihydrotestosterone is thought to be the result of elevated intracellular bonding based on receptor increase. The increase in receptors is stimulated by the elevation of the estrogen levels relative to androgen levels which decrease with age. The urological symptoms consist in an elevated frequency of miction due to elevated residual urine, which bothers the patients especially during the night hours. This is accompanied by a weak flow of urine, a time-delayed start of miction, and repeated infections of the bladder and kidneys.

Lower urinary tract symptoms (LUTS) are a common problem affecting older men. Symptoms can be categorised into filling symptoms (previously called irritative) and voiding symptoms (previously called obstructive). Symptoms are non-specific and large studies of patients have failed to show any correlation between lower urinary tract symptoms and a specific diagnosis. LUTS are common and not necessarily a reason for suspecting prostate cancer.

Prostate cancer is a serious condition that affects increasing numbers of men worldwide. About one-third of all men have at least some cancerous prostatic cells at age 50, with the incidence increasing to as many as 90 percent of men at age 90. In the United States alone, about 40,000 men die each year from prostate cancer.

Endometriosis is one of the most frequently encountered pathologies diagnosed amongst gynecological patients. For example, between 10% and 25% of women presenting with gynecological symptoms in UK and in the USA are affected. Endometriosis is the ectopic presence of endometrial type glands and stroma in sites which are outside of the uterus. This ectopic occurrence of endometrial tissue frequently forms cysts containing altered blood. The condition results in debilitating pain for millions of women worldwide and particularly occurs in conjunction with the monthly proliferation of endometrial tissue. Endometriosis is frequently a lifelong condition.

Ovarian cancer is often called the silent killer because many times there are no symptoms until the disease has progressed to an advanced stage. One-third of American women will get some form of cancer in their lifetime and approximately 1.4% of those cases will be cancer involving one or both ovaries.

Dysfunctional (or abnormal) uterine bleeding (DUB) is a problem that often affects women as they start to get periods and as they get closer to menopause, although any woman who menstruates can experience DUB. The main symptoms are prolonged and/or irregular menstrual bleeding. The bleeding may be irregular spotting during the cycle, but sometimes the bleeding is so heavy that a woman can't participate in her normal day-to-day activities, such as work and exercise.

The discovery and characterization of LHRH (luteinizing hormone-releasing hormone, also referred to as GnRH) as the first mediator in the hypothalamic-pituitary-gonadal axis has opened up new possibilities for the treatment of sex hormone-dependent conditions such as prostate cancer, breast cancer, endometriosis, uterine leiomyoma (fibroids), ovarian cancer, prostatic hyperplasia, sexual dysfunction, assisted reproductive therapy, and precocious puberty (The Lancet 358:1793-1803, 2001; Mol. Cell. Endo. 166:9-14, 2000).

LHRH plays a key role in the control of mammalian reproduction. LHRH is secreted in a pulsatile manner from hypothalamic neurons and regulates the synthesis and secretion of the gonadotropins (follicle stimulating hormone (FSH) and luteinizing hormone (LH)) from the anterior pituitary gonadotropes. This action of LHRH is achieved by binding to and activation of its high affinity receptors located on the plasma membrane of the gonadotropes. LHRH or LHRH-like immunoreactivity and LHRH mRNA have been reported in human ovary, breast, testes, pituitary, as well as in a number of human tumors and tumor cell lines. The existence of high affinity LHRH receptor and its mRNA has been shown using LHRH superagonists in radioreceptor assays and reverse transcriptase-polymerase chain reaction techniques in human ovary, breast, prostate, testes, and in a number of hormone-related tumors including breast tumor, prostate tumor, endometrial tumor, ovarian tumor, kidney tumor, pancreas tumor, and tumor cell lines. Nucleotide sequences of LHRH receptors isolated from a breast tumor cell line (MCF-7) and from an ovarian tumor showed 100% homology with the human pituitary receptor.

The cloning and sequencing of the LHRH receptor from human pituitary has made it possible to investigate the expression of LHRH and LHRH receptor genes in human non-reproductive tissues. In human, LHRH receptor mRNA is also expressed in liver, heart, skeletal muscle, and kidney (Cancer Letters 98 (1995) 57-62).

Because desensitization of the LHRH receptor is very effective in blocking LH/FSH release, several analogs of LHRH have been approved for the treatment of sex hormone dependent disorders in humans.

Several studies have shown that treatments with the combination of a LHRH analog and a further active compound are more effective than treatments with a LHRH analog alone.

WO9014839 describes the use of gonadotropin releasing hormone analogues in combination with gonadotropins and growth hormone for infertility treatment. WO92/16233 describes the combined use of an inhibitor of 5-alpha-reductase and an anti-androgen for the treatment of prostate cancer. The combination of Finasteride with an anti-androgen, e.g. Flutamide, is also taught. The use of a composition of various LHRH agonists and of an anti-androgen for treating BPH is also suggested in the US patent US 4,472,382. DE 19604231 discloses the use of combinations of LH-RH analogs and antiestrogens for the treatment of gynecological disorders. DE19825591 refers to pharmaceutical combinations which compensate an abs. or relative testosterone deficit with simultaneous prophylaxis against formation of benign prostatic hyperplasia or prostate carcinoma. The combinations of the invention contain a natural or synthetic androgen in combination with a gonadotropin-releasing hormone analog. WO9917767 describes methods of treating and preventing endometriosis with phytoestrogens in combination with GnRH analogs, androgens, progestins, estrogens or any combination thereof. WO2001060365 discloses methods of Treatment or prevention of prostate cancer with a COX-2 selective inhibiting drug in combination with LHRH analogs. Palomba et al. (Human Reproduction (2002), 17(12), 3213-3219) describe a combined administration of GnRH analogue and raloxifene in the treatment of uterine leiomyomas. WO2002056903 refers to methods for treating hormone associated conditions using a combination of LHRH antagonists and specific estrogen receptor modulators. WO2002036144 relates to GnRH analogues for treatment of urinary incontinence and other side effects associated with ovariectomy or reproductive senescence in humans and dogs in combination with an additional active ingredient such as: an estrogenic agent, a partial estrogenic agent, a progestational agent, an a-adrenergic agonist, a b-adrenergic receptor blocking agent, a cholinergic receptor blocking compd., a cholinergic receptor stimulating drug, a smooth muscle relaxant, a nitric oxide synthase substrate or a nitric oxide donor. WO2004087190 discloses a method for the treatment of advanced prostate cancer by administering an androgen-suppressing LH releasing hormone agonist analog and calcitriol. WO2005046691 describes a combination of a farnesyl transferase inhibitor with an antihormonal agent for the treatment of breast cancer. WO2006106311 relates to combination treatment methods using GnRH and/or GnRH analog and prostaglandin synthesis inhibitor and/or prostaglandin receptor antagonist for the treatment of sex-hormone dependent diseases. Torrisi et al. (British Journal of Cancer (2007), 97(6), 802-808) describe letrozole and GnRH analogue as primary therapy in premenopausal women with ER and PgR positive locally advanced operable breast cancer.

An alternative approach to treat sex hormone dependent disorders is the use of PDE5 inhibitors.

DE19825591 refers to Pharmaceutical combinations which compensate an abs. or relative testosterone deficit with simultaneous prophylaxis against formation of benign prostatic hyperplasia or prostate carcinoma. The combinations of the invention contain a natural or synthetic androgen in combination with a phosphodiesterase inhibitor. WO2003028730 discloses combinations containing a phosphodiesterase inhibitor and i) an anti-diabetic agent; (ii) HMG-Co-A reductase inhibitors; (iii) an antihypertensive agent; or (iv) a serotonin reuptake inhibitor (SSRI) for the treatment of sexual dysfunction, hyperglycemia, hyperinsulinemia, hyperlipidemia, hypertriglyceridemia, diabetes, insulin resistance, impaired glucose metabolism, conditions of impaired glucose tolerance (IGT), conditions of impaired fasting plasma glucose, obesity, diabetic retinopathy, diabetic nephropathy, glomerulosclerosis, diabetic neuropathy, syndrome X, erectile dysfunction, coronary heart disease, hypertension, esp. ISH, angina pectoris, myocardial infarction, stroke, vascular restenosis, endothelial dysfunction, impaired vascular compliance or congestive heart failure. WO2004087211 relates to methods for the treatment of infertility with inhibitors of phosphodiesterases (PDE) in conjunction with gonadotropins. US2005222014 relates to Phosphodiesterase V inhibitor combination with melanocortin 3 and/or 4 receptor agonist for treatment of sexual dysfunction. WO2007039075 describes the use of PDE-5 inhibitors in combination with PDE-4 inhibitors for the treatment of urological disorders benign prostate hyperplasia (BPH), lower urinary tract symptoms (LUTS) and in particular irritative symptoms caused by BPH-induced bladder outlet obstruction (BOO). WO2007072156 refers to a Pharmaceutical combination of a PDE5 inhibitor and a 5 alpha reductase inhibitor for the treatment of lower urinary tract symptoms (LUTS), such as urgency, frequency, nocturia and urge incontinence. WO2007072169 relates to the combined use of a phosphodiesterase 5 inhibitor and a muscarinic antagonist in the treatment of lower urinary tract symptoms (LUTS). WO2008054215 discloses the use of 3-alpha- androstanediol in combination with a 5-HT1A agonist and a type 5 phosphodiesterase (PDE5) inhibitor for the treatment of male and/or female sexual dysfunction. WO2008138483 describes combinations of a soluble guanylate cyclase stimulator or activator with a PDE5 inhibitor for the treatment of urological disorders comprising benign prostate syndrome (BPS), benign prostate hyperplasia (BPH), benign prostate enlargement (BPE), bladder outlet obstruction (BOO), lower urinary tract symptoms (LUTS), genitourinary disorders comprising neurogenic bladder syndrome, such as overactive bladder (OAB) and interstitial cystitis (IC) urinary incontinence (UI), like mixed-, urge-, stress-, or overflow incontinence (MUI, UUI, SUI, OUI) and pelvic pain.

Thus, it is well established in the prior art that sex hormone dependent disorders can be treated with LHRH analogs or PDE5 inhibitors in combination with further active compounds. However, none of these means is considered entirely satisfactory at the present time. While significant strides have been made in this field, there remains a need for methods of treating sex hormone related conditions, in which the therapeutic effect is improved and negative hormone withdrawal symptoms are prevented. The present invention fulfills this need, and provides other related advantages.

### DESCRIPTION OF THE INVENTION

The problem of the present invention is to provide novel methods of treatment of sex hormone dependent disorders, in particular BPH and LUTS, in which the therapeutic effect is improved and negative hormone withdrawal symptoms are prevented.

The problem of the present invention has surprisingly been solved in one aspect by providing a method of treating or preventing a sex hormone dependent disorder in a mammal, comprising administering to the mammal at least one LHRH antagonist and at least one PDE V inhibitor.

None of the documents of the prior art discloses or suggests the combination of a LHRH antagonist and a PDE V inhibitor for the treatment or prevention of a sex hormone dependent disorder in a mammal.

The mammal to be treated may be any mammal such as cats, dogs, cows, sheep, horses and the like. It is particularly preferred if the mammal is a human. The mammal may be female or male.

The term "sex hormone dependent disorder" as used in the present invention refers to BPH, LUTS, breast cancer, prostate cancer, ovarian cancer, uterine cancer endometriosis, premenstrual syndrome, polycystic ovary disease, postmenopausal syndrome, uterus myoma, infertility, erectile dysfunction or female sexual dysfunction. In a preferred embodiment of the present invention, it refers to BPH or LUTS.

The term "LHRH antagonist" as used in the present invention means any LHRH analogue, whether peptide or non-peptide, except for Cetrorelix, which inhibits, reduces or prevents signaling of the GnRH receptor. Inhibition, reduction or prevention of LHRH-R signaling can readily be determined by a person of skill in the art.

Current LHRH antagonists are LHRH-like decapeptides which are generally administered by nasal sprays, intravenously or subcutaneously presumably because of negligible oral activity. By LHRH we mean the decapeptide pGlu-His-Trp-Ser-Tyr-Gly-Leu-Arg-Pro-Gly- NH2 in which the pGlu is pyroglutamate. Peptidic LHRH antagonists have amino acid substitutions usually at positions one, two, three, six and ten. Non-peptide LHRH antagonists have been described in PCT application 2008/107446. Non-peptide LHRH antagonists offer the possible advantage of oral administration. The most preferred LHRH antagonist is Ozarelix. Other suitable LHRH antagonists include Teverelix, Degarelix, Ganirelix, Abarelix, Antarelix, Detirelix, Ramorelix, Antide, Azaline B, A-75998, A-84861, RS-68439, Org30850, Org30276, ORF18260, Acycline, TAK-013, TAK-810, NBI-42902, NBI-56418, T-98475, AG-045572, CAS-874112-24-0, CAS-874112-16-0, CAS-1056021-27-2, CAS-1056021-26-1, CAS-1056021-45-4 and CAS-1056022-61-7.

The term "PDE V inhibitor" as used in the present invention means a compound that inhibits cGMP hydrolysis by phosphodiesterase-5. PDE V inhibitors preferably reduce PDE V enzymatic activity by at least 5%. Methods for assaying the activity of a PDE V inhibitor are known in the art. The most preferred PDE V inhibitors are Sildenafil and Tadalafil. Other suitable PDE V inhibitors are Vardenafil, Mirodenafil, Thioquinapiperifil, Zaprinast, Udenafil, Avanafil, Dasantafil, BMS 341400, BMS 281384, BMS 263504, LAS 34179, LAS 30904, LAS 34837, AWD 12-250, FR 189318, FR 229934, FR 226807, EMD 82639, UK 369003, UK 114502, UK 114542, EMR 62-203, QAD 171A, SR 265579 and SLx 2101.

The LHRH antagonist may be administered at the same time as the PDE V inhibitor or it may be administered before or after. It will be appreciated that the invention includes the administration to the mammal of one or more LHRH antagonists and one or more PDE V inhibitors.

In a most preferred embodiment, the at least one LHRH antagonist is Ozarelix and the at least one PDE V inhibitor is Sildenafil or Tadalafil.

The present invention further relates to the use of a combination of at least one LHRH antagonist with at least one PDE V inhibitor in the manufacture of a medicament for treating or preventing a sex hormone dependent disorder in a mammal.

Finally, the present invention refers to a pharmaceutical composition comprising a therapeutically effective amount of at least one LHRH antagonist, a therapeutically effective amount of at least one PDE V inhibitor, customary pharmaceutical carriers, excipients and/or diluents, for use in the treatment or prevention of a sex hormone dependent disorder in a mammal.

The amounts of the therapeutic agents, i.e. the at least one LHRH antagonist and the at least one PDE V inhibitor which are administered are amounts which are effective to ameliorate the disorder condition of the mammal to a useful extent or are amounts which provide the desired clinical effect according to the use to which the therapeutic agents are put. This may be determined by the clinician. The amount of therapeutic agents administered to the mammal may be different according to the clinical application in question, and this may be determined by the clinician. Similarly, the treatment regime (e.g. doses, timings of administration and so forth) may be determined by the clinician.

The therapeutic agents used in the methods of the invention may be administered using any suitable means or route of administration. Conveniently, the known means and routes of administration are used for each of the different therapeutic agents.

It will be appreciated that the at least one LHRH antagonist may be administered by one route and the at least one PDE V inhibitor may be administered by another route; alternatively, they may both be administered by the same route.

### EXAMPLE

### Ozarelix + sildenafil

Treatment course with oxarelix will include one IM injection of 15mg ozarelix at the beginning of the treatment course and one additional IM injection of 15mg ozarelix 14 days later. This treatment course with oxarelix will be repeated each six months.

### COMBINED WITH

50 mg sildenafil per os once daily. Dosing of sildenafil may be escalated to 100mg once daily two weeks after the beginning of the treatment.

## Claims

1. A method of treating or preventing a sex hormone dependent disorder in a mammal, comprising administering to the mammal at least one LHRH antagonist except for Cetrorelix and at least one PDE V inhibitor.

2. The method according to claim 1, wherein the sex hormone dependent disorder is BPH, LUTS, breast cancer, prostate cancer, ovarian cancer, uterine cancer, endometriosis, uterus myoma, polycystic ovary disease, premenstrual syndrome, postmenopausal syndrome, infertility, erectile dysfunction or female sexual dysfunction.

3. The method according to any one of claim 1 or 2, wherein the sex hormone dependent disorder is BPH or LUTS.

4. The method according to any one of claims 1 to 3, wherein the at least one LHRH antagonist is selected from the group consisting of: Ozarelix, Teverelix, Degarelix, Ganirelix, Abarelix, Antarelix, Detirelix, Ramorelix, Antide, Azaline B, A-75998, A-84861, RS-68439, Org30850, Org30276, ORF18260, Acycline, TAK-013, TAK-810, NBI-42902, NBI-56418, T-98475, AG-045572, CAS-874112-24-0, CAS-874112-16-0, CAS-1056021-27-2, CAS-1056021-26-1, CAS-1056021-45-4 and CAS-1056022-61-7.

5. The method according to any one of claims 1 to 4, wherein the at least one PDE V inhibitor is selected from the group consisting of: Sildenafil, Vardenafil, Tadalafil, Mirodenafil, Thioquinapiperifil, Zaprinast, Udenafil, Avanafil, Dasantafil, BMS 341400, BMS 281384, BMS 263504, LAS 34179, LAS 30904, LAS 34837, AWD 12-250, FR 189318, FR 229934, FR 226807, EMD 82639, UK 369003, UK 114502, UK 114542, EMR 62-203, QAD 171A, SR 265579 and SLx2101.

6. The method according to any one of claims 1 to 5, wherein the at least one LHRH antagonist is Ozarelix.

7. The method according to any one of claims 1 to 6, wherein the at least one PDE V inhibitor is selected from the group consisting of: Sildenafil and Tadalafil.

8. The method according to any one of claims 1 to 7, wherein the at least one LHRH antagonist is Ozarelix and the at least one PDE V inhibitor is Sildenafil or Tadalafil.

9. Use of a combination of at least one LHRH antagonist except for Cetrorelix with at least one PDE V inhibitor in the manufacture of a medicament for treating or preventing a sex hormone dependent disorder in a mammal.

10. Use according to claim 9, wherein the sex hormone dependent disorder is BPH, LUTS, breast cancer, prostate cancer, ovarian cancer, uterine cancer, endometriosis, uterus myoma, polycystic ovary disease, premenstrual syndrome, postmenopausal syndrome, infertility, erectile dysfunction or female sexual dysfunction.

11. Use according to any one of claim 9 or 10, wherein the sex hormone dependent disorder is BPH or LUTS.

12. Use according to any one of claims 9 to 11, wherein the at least one LHRH antagonist is selected from the group consisting of: Ozarelix, Teverelix, Degarelix, Ganirelix, Abarelix, Antarelix, Detirelix, Ramorelix, Antide, Azaline B, A-75998, A-84861, RS-68439, Org30850, Org30276, ORF18260, Acycline, TAK-013, TAK-810, NBI-42902, NBI-56418, T-98475, AG-045572, CAS-874112-24-0, CAS-874112-16-0, CAS-1056021-27-2, CAS-1056021-26-1, CAS-1056021-45-4 and CAS-1056022-61-7.

13. Use according to any one of claims 9 to 12, wherein the at least one PDE V inhibitor is selected from the group consisting of: Sildenafil, Vardenafil, Tadalafil, Mirodenafil, Thioquinapiperifil, Zaprinast, Udenafil, Avanafil, Dasantafil, BMS 341400, BMS 281384, BMS 263504, LAS 34179, LAS 30904, LAS 34837, AWD 12-250, FR 189318, FR 229934, FR 226807, EMD 82639, UK 369003, UK 114502, UK 114542, EMR 62-203, QAD 171A, SR 265579 and SLx2101.

14. Use according to any one of claims 9 to 13, wherein the at least one LHRH antagonist is Ozarelix.

15. Use according to any one of claims 9 to 14, wherein the at least one PDE V inhibitor is selected from the group consisting of: Sildenafil and Tadalafil.

16. Use according to any one of claims 9 to 15, wherein the at least one LHRH antagonist is Ozarelix and the at least one PDE V inhibitor is Sildenafil or Tadalafil.

17. A pharmaceutical composition comprising a therapeutically effective amount of at least one LHRH antagonist except for Cetrorelix, a therapeutically effective amount of at least one PDE V inhibitor, customary pharmaceutical carriers, excipients and/or diluents, for use in the treatment or prevention of a sex hormone dependent disorder in a mammal.

18. The pharmaceutical composition according to claim 17, wherein the sex hormone dependent disorder is BPH, LUTS, breast cancer, prostate cancer, ovarian cancer, uterine cancer, endometriosis, uterus myoma, polycystic ovary disease, premenstrual syndrome, postmenopausal syndrome, infertility, erectile dysfunction or female sexual dysfunction.

19. The pharmaceutical composition according to any one of claim 17 or 18, wherein the sex hormone dependent disorder is BPH or LUTS.

20. The pharmaceutical composition according to any one of claims 17 to 19, wherein the at least one LHRH antagonist is selected from the group consisting of: Ozarelix, Teverelix, Degarelix, Ganirelix, Abarelix, Antarelix, Detirelix, Ramorelix, Antide, Azaline B, A-75998, A-84861, RS-68439, Org30850, Org30276, ORF18260, Acycline, TAK-013, TAK-810, NBI-42902, NBI-56418, T-98475, AG-045572, CAS-874112-24-0, CAS-874112-16-0, CAS-1056021-27-2, CAS-1056021-26-1, CAS-1056021-45-4 and CAS-1056022-61-7.

21. The pharmaceutical composition according to any one of claims 17 to 20, wherein the at least one PDE V inhibitor is selected from the group consisting of: Sildenafil, Vardenafil, Tadalafil, Mirodenafil, Thioquinapiperifil, Zaprinast, Udenafil, Avanafil, Dasantafil, BMS 341400, BMS 281384, BMS 263504, LAS 34179, LAS 30904, LAS 34837, AWD 12-250, FR 189318, FR 229934, FR 226807, EMD 82639, UK 369003, UK 114502, UK 114542, EMR 62-203, QAD 171A, SR 265579 and SLx 2101.

22. The pharmaceutical composition according to any one of claims 17 to 21, wherein the at least one LHRH antagonist is Ozarelix.

23. The pharmaceutical composition according to any one of claims 17 to 22, wherein the at least one PDE V inhibitor is selected from the group consisting of: Sildenafil and Tadalafil.

24. The pharmaceutical composition according to any one of claims 17 to 23, wherein the at least one LHRH antagonist is Ozarelix and the at least one PDE V inhibitor is Sildenafil or Tadalafil.
